# EUROPEAN PATENT APPLICATION

(11) **EP 2 420 182 A1**
(43) Date of publication of application: **22.02.2012**
(21) Application number: 10764375.1
(22) Date of filing: 06.04.2010
(51) Int. Cl.: A61B 3/14, G06T 1/00

(54) **IMAGE PROCESSING METHOD AND IMAGE PROCESSING APPARATUS**

(30) Priority: 15.04.2009 JP 2009098542
(71) Applicant: Kowa Company, Ltd., Nagoya-shi, Aichi-ken 460-8625 (JP)
(72) Inventor: MIZUKUSA Yutaka, Hamamatsu-shi Shizuoka 431-2103 (JP); NAKAGAWA Toshiaki, Hamamatsu-shi Shizuoka 431-2103 (JP)
(74) Representative: Kronthaler, Wolfgang N.K.
(86) International application number: PCT/JP2010/056193
(87) International publication number: WO 2010/119790

(57) **Abstract**

The ocular fundus of an eye under examination is stereo-photographed via an ocular fundus photographing optical system with a predetermined parallax (S100). The photographed stereo ocular fundus images are subjected to color separation (S101) when a process of measuring the three-dimensional shape of the ocular fundus of the eye under examination is to be performed using left and right parallax images obtained. A depth information measurement process to derive depth information of a specific ocular fundus region is carried out on the respective stereo ocular fundus images of different wavelengths obtained by the color separation (S103), and a thickness information measurement process is carried out to derive, as thickness information for specific ocular fundus tissue, a difference of depth information obtained respectively from stereo ocular fundus images of different wavelength components in the depth information measurement process (S104). Additionally, a spatial frequency filtering process is carried out for an image of a specific frequency component (S103).

## Description

### Technical Field

The present invention relates to an image processing method and an image processing apparatus for outputting, for display, ocular fundus images of an eye under examination.

### Background Art

There are known in the prior art image processing apparatuses such as fundus cameras for stereo-photographing the ocular fundus of an eye under examination in order to ascertain the ocular fundus condition of the eye under examination for the purpose of diagnosing glaucoma or the like. Stereo-photographing of the ocular fundus of an eye under examination is performed by moving a single aperture within an optical system of a fundus camera to different positions that are decentered to left and right (or up and down) from the optical axis while carrying out photographing at the respective aperture positions. From the left and right stereo images, depth information can be derived at regions corresponding to the left and right images. This, for example, allows a stereoscopic shape model of the ocular fundus to be created.

It has also been attempted to carry out three-dimensional analysis for different spectral images (e.g., R, G, and B color images) that are obtained by color photographing (Patent Document 1 and Patent Document 2 below). Patent Document 1 discloses a technique for carrying out three-dimensional analysis for each color (layer) of R-, G-, and B-specific stereo images and synthesizing depth information of the ocular fundus in every spectral image to calculate the three-dimensional shape of the ocular fundus.

Patent Document 2 discloses a technique in which a stereo fundus camera for photographing the ocular fundus using a stereo optical system is provided with optical separation means that optically separate wavelengths of light simultaneously guided from layers of the ocular fundus to simultaneously capture images of the layers of the ocular fundus, and three-dimensional analysis of each color (layer) of R-, G-, and B-specific stereo images is carried out so that sectional differences in stereo images obtained, for example, from two specific spectra can be grasped numerically to provide the thickness of the fibrous layer of the retina.

This prior art is based on the idea that measuring the thickness of the fibrous layer of the retina is useful in terms of diagnosing glaucoma and grasping its pathology.

### Prior Art Documents

### Patent Documents

Patent Document 1: Japanese Laid-open Patent Application 2000-245700
Patent Document 2: Japanese Patent No. 2919855

### Summary of Invention

### Problems to be Solved

Measuring the thickness of the retinal layer of an eye under examination as well as creation of retinal thickness maps from ocular fundus images, for example, can already be accomplished with OCT (an apparatus for measuring the ocular fundus using an optical coherence tomography optical system) or devices that use a polarized scan laser beam to measure the nerve fibrous layer of the retina. However, all of these methods require expensive specialized devices, and it has been necessary to photograph the ocular fundus separately.

For example, if retina thickness information could be measured using stereo fundus camera hardware, it would be preferably carried out to photograph the ocular fundus and measure retina thickness information with a simple and inexpensive arrangement. However, when attempted to perform color separations and measure retina thickness using a stereo fundus camera having a configuration such as that disclosed in the aforedescribed Patent Document 1 or 2, there arises the problem that reflected light enters from a different layer into each color image, thus making correct measurement impossible at the region thereof. In particular, this problem tends to occur frequently in the red component (R component) of longer wavelength that represents light reflected from a region of the choroidal tissue deeper than the retinal tissue, and the signals from the deep layer part and the surface layer part are intermixed in the red component image. This presents the problem of an inability to obtain sufficient measurement accuracy.

In view of the foregoing problem, it is an object of the present invention to accurately measure information relating to tissue of the ocular fundus, in particular to the thickness of the retina, from ocular fundus images obtained by stereo-photographing with light of different wavelengths.

### Means for Solving the Problems

In order to solve the problem, the present invention provides an image processing method in which an ocular fundus of an eye under examination is stereo-photographed with a predetermined parallax via an ocular fundus photographing optical system to provide left and right parallax images, which are used for processes of measuring a three-dimensional shape of the ocular fundus of the eye under examination, comprising: subjecting the photographed stereo ocular fundus images to color separation; performing a depth information measurement process in which depth information at a specific ocular fundus region is derived for each of the stereo ocular fundus images of different wavelength components obtained by the color separation, and performing a thickness information measurement process in which a difference in the depth information obtained respectively in the depth information measurement process from the stereo ocular fundus images of different wavelength components is derived as thickness information for specific ocular fundus tissue.

### Effect of the Invention

According to the aforedescribed configuration, information relating to the tissue of the ocular fundus, in particular to the thickness of the retina, can be measured accurately from fundus images obtained by stereo-photographing with light of different wavelengths.

### Brief Description of Drawings

FIG. 1 is a block diagram showing a configuration of an image processing system employing the present invention;
FIG. 2a is a flowchart showing the flow of image processing in the image processing system employing the present invention;
FIG. 2b is a flowchart showing the flow of image processing in the image processing system employing the present invention;
FIG. 3a is a flowchart showing the flow of image processing in the image processing system employing the present invention;
FIG. 3b is a graph describing the principle of image processing in the image processing system employing the present invention;
FIG. 4 is a flowchart showing the flow of still another image processing in the image processing system employing the present invention;
FIG. 5 is an illustrative diagram showing stereo ocular fundus images captured by the image processing system employing the present invention;
FIG. 6 is an illustrative diagram showing image processing in the image processing system employing the present invention;
FIG. 7 is an illustrative diagram showing image processing in the image processing system employing the present invention;
FIG. 8 is an illustrative diagram showing an example of display and output in the image processing system employing the present invention; and
FIG. 9 is an illustrative diagram showing an example of display and output in the image processing system employing the present invention.

### Mode of Carrying Out the Invention

By way of an example of the best mode for carrying out the invention, embodiments will be described below that relate to an ophthalmic measurement apparatus in which the ocular fundus of an eye under examination is stereo-photographed via a stereo-photographic optical system and a three-dimensional measurement process is carried out for the obtained data of captured images.

### Embodiment 1

### <System Configuration>

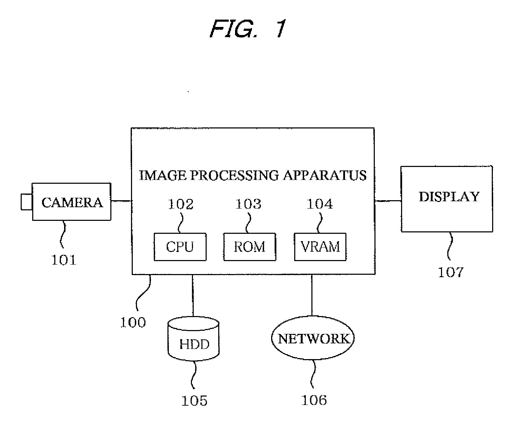

FIG. 1 shows a configuration of an ophthalmic measurement apparatus employing the present invention. In FIG. 1, reference numeral 101 denotes a fundus photographing camera comprising a fundus camera or the like provided with a mechanism for photographing the ocular fundus of an eye under examination (not shown) under predetermined photographic conditions, including, for example, an alignment mechanism for determining photographing distance. The camera 101 has an imaging element such as, for example, a three-plate CCD, CMOS sensor capable of carrying out color photographing. The camera outputs color fundus image data of a photographed eye under examination as digital data to an image processing apparatus. In cases where the image signal outputted by the camera 101 has a format such as YUV (YPbPr or YCbCr), a process for separating colors into image data of different spectra such as RGB image data is carried out by a color separation process such as that described below. Such a color separation process will be necessary, for example, in cases where the camera 101 outputs images in the JPEG (or MPEG) format.

An image processing apparatus 100 is constituted, for example, using hardware such as a PC. The image processing apparatus 100 carries out control of the overall system, and includes a CPU 102 constituting principal image processing means for carrying out image processing to be described later. It is needless to say that the image processing apparatus 100 could be constituted by specialized hardware integrally constituted with the camera 101.

Image processing to be described below is executed using a VRAM (image memory) 104 as the work area. In addition to this, as memory used for system control or purposes other than image processing, the system may be furnished with memory constituted by dynamic RAM or the like. A program for the CPU 102 to carry out image processing as described later is stored in a ROM 103 or an HDD 105.

The HDD 105 is also used for storing image data from photographing of eyes under examination, numeric data such as measurement results, output image data generated by image processing as described later, and the like.

A display 107 composed of an LCD, EL panel, CRT, or the like is connected as display output means to the image processing apparatus 100. Displayed on the display 107 are output images, user interface screens for controlling image processing performed by the image processing apparatus 100, and the like. For the purposes of image display and carrying out control of the overall system, the image processing apparatus 100 is assumed to be provided with user interface means comprising a keyboard, and a mouse or another pointing device (not shown).

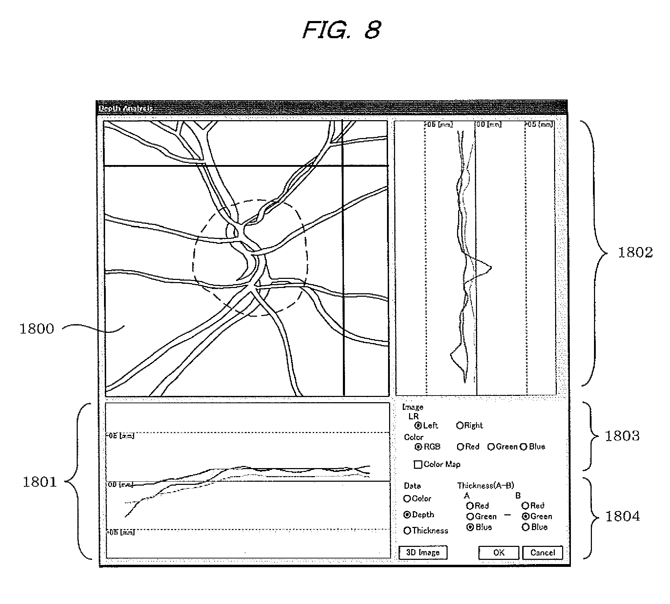

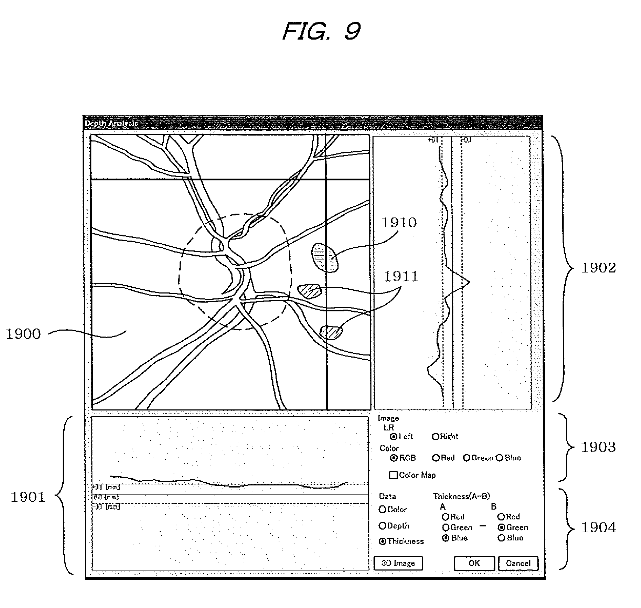

On the basis of the image processing to be described later, the image processing apparatus 100 generates image data processed such that the technician is readily able to carry out an evaluation in relation to the ocular fundus of an eye under examination, in particular to the thickness of the retinal layer, and the image data is outputted to the display 107 (FIGS. 8, 9).

A network 106 is connected to the image processing apparatus 100 via a network interface (not shown). The image processing apparatus 100 outputs the image data from photographing of the eye under examination, numeric data such as measurement results, output image data generated by image processing to be described later, and the like to an external computer, another image processing apparatus, an ophthalmic measurement device, or the like.

### <Image processing>

A feature of the present embodiment is that, using image data (e.g., RGB images) obtained at different spectra such as data of RGB images color-photographed by the camera 101, three-dimensional information, in particular a depth distribution of the ocular fundus of the eye under examination is derived for every color image (i.e., color-separated images).

For example, in the case of an RGB image, basically, the R component can be treated as reflected light containing plenty of information from a relatively deep part of the retina such as the choroid; the G component as reflected light containing plenty of information from the pigment epithelium of the retina; and the B component as reflected light containing plenty of information from the retina surface. Consequently, any two of depth information obtained from these color components are selected to provide the distance (thickness) between layers that are well-reflective of image information thereof. For example, theoretically, the difference of depth information obtained from the R component and depth information obtained from the G component is calculated to provide a distance which can be determined as thickness from the choroid to the pigment epithelium of the retina.

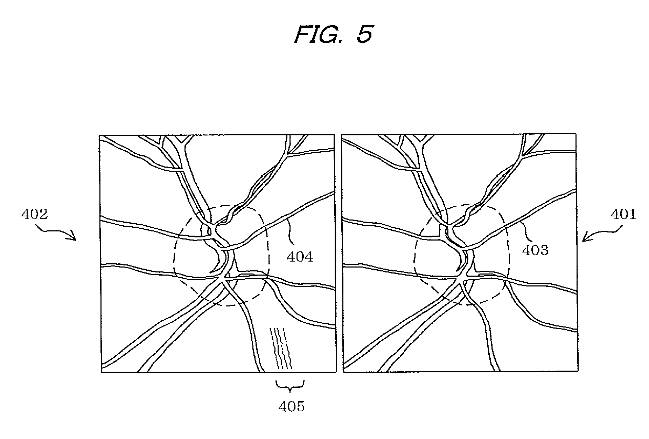

However, the R component of an ocular fundus image may sometimes contain reflected light from the retina surface in proximity to the mid- to high range of the spatial frequency thereof. FIG. 5 shows left and right fundus images 402, 401 having been stereo-photographed by the camera 101. In the drawing, the regions to the inside of the broken lines show "optic disk" regions which are photographed relatively brightly, while reference numerals 403 and 404 show blood vessels of the ocular fundus. As shown only in a very small part at the bottom of the left image 402 in FIG. 5, the image contains reflected light from the retina surface, which is outputted because of its existence in proximity to the mid- to high range of the spatial frequency of the R component and the B component.

As described above, due to mixing of reflected light (405) from a different layer as shown in FIG. 5, misleading depth data may occur, and inaccurate or meaningless thickness information may be outputted. For example, the R component, which as noted above is reflective of three-dimensional information from the deep tissue layer, does contain reflected light from the retina surface as well. Depth information measured from the left and right stereo images is depth information of a mix of the two layers, so that, in a case where, for example, a difference is computed from depth information obtained from the G component which is reflective of three-dimensional information from the middle tissue layer, a range may occur in which a negative number is outputted as thickness information of the tissue.

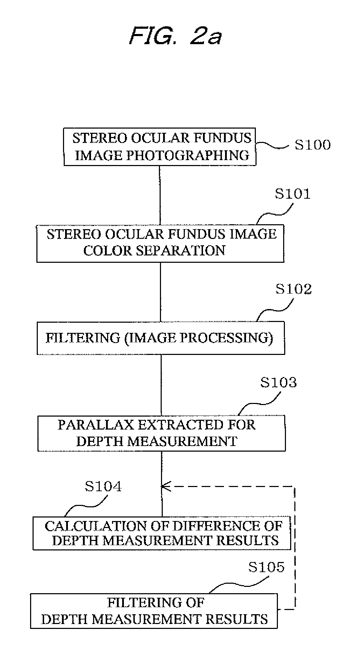

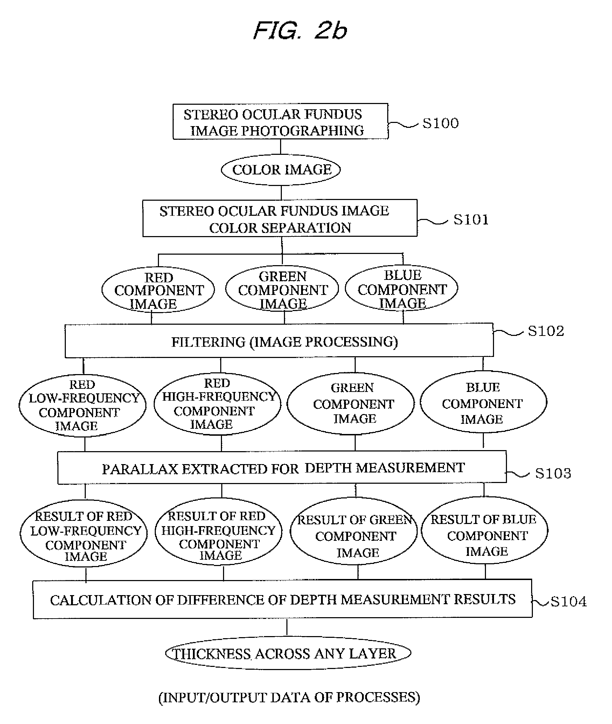

To solve problems such as the above, in the present embodiment, image processing is carried out as shown in FIGS. 2a and 2b. FIGS. 2a and 2b show respectively different flows of an image processing routine in the image processing system of the present embodiment. A program for the CPU 102 to carry out image processing as the principal processor of the image processing apparatus 100 is stored in the ROM 103 or the HDD 105.

In the image processing of FIGS. 2a and 2b, according to the present embodiment, images that are obtained by color stereo-photographing and undergoes color separation to the RGB color components are used respectively to provide stereo images of each color component, and depth information of tissue corresponding, for example, to the choroid or pigment epithelium of the retina is derived therefrom to provide the difference thereof as representative of retina thickness, wherein a filtering process relating to spatial frequency of a specific color component is carried out in order to reduce or eliminate the effect of measurement errors occurring due to the noise component (FIG. 5) as described above.

FIGS. 2a and 2b show a flow of a program for the CPU 102 to carry out image processing as the principal processor of the image processing apparatus 100. FIGS. 2b shows the same process as FIG. 2a, but explicitly shows how the color component data is processed. In FIGS. 2a and 2b, identical process steps are assigned identical step numbers.

In Step S100 of FIGS. 2a and 2b, an ocular fundus image of an eye under examination is obtained by color stereo-photographing using the camera 101, and in Step S101, a color stereo ocular fundus image signal outputted from the camera 101 undergoes color separation to an RGB image signal, which is stored in the VRAM (video memory) 104. In cases where a native RGB signal is outputted from the camera 101, it will be sufficient to store the RGB data thereof in the VRAM (video memory) 104. However, where the camera 101 uses an image format different from the RGB format, such as a specific YUV format, the color separation process of Step S101 becomes necessary.

As shown by Steps S100 to S101 of FIG. 2b, the color stereo images outputted from the camera 101 undergo color separation into red (R) component image data, green (G) component image data, and blue (B) component image data.

Next, in Step S102, a specific filtering process is carried out on the obtained red (R) component image data, green (G) component image data, and blue (B) component image data. Optionally, this process may be omitted through a user setting (see FIG. 8 described below).

As shown in FIG. 2b, the filtering process is carried out on the respective image data. In the present embodiment, among the red (R) component image data, green (G) component image data, and blue (B) component image data of the stereo images, the red (R) component image data undergoes extraction of images of low-frequency and high-frequency components of spatial frequency, respectively. As a result of the filtering process of Step S102 there is obtained red (R) low-frequency component image data, red (R) high-frequency component image data, green (G) component image data, and blue (B) component image data of the stereo images.

In Step S103, a parallax is extracted from the left and right stereo images of the respective components of the red (R) low-frequency component image data, the red (R) high-frequency component image data, the green (G) component image data, and the blue (B) component image data, and depth information is measured for corresponding pixels of the left and right images. Here, the method by which depth information is measured for corresponding pixels from the left and right images is a known method, and a detailed description is omitted here.

As shown in FIG. 2b, the depth measurement process of Step S103 allows depth measurement results to be respectively obtained for the red (R) low-frequency component image data, the red (R) high-frequency component image data, the green (G) component image data, and the blue (B) component image data. If necessary, as shown by Step S105 in FIG. 2a, a specific filtering process can be carried out on the respective depth measurement results of the red (R) low-frequency component image data, the red (R) high-frequency component image data, the green (G) component image data, and the blue (B) component image data. A smoothing filter or a median filter may be considered for use as the filtering process.

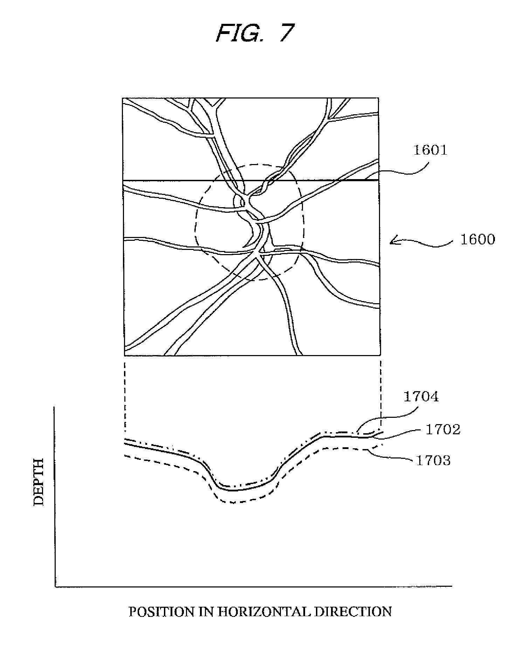

Then, in Step S104, the difference between two specific depth measurement results among these is calculated, and the differential thereof can be outputted as thickness across any layer. For example, performing the above-described filtering causes the curve of depth information obtained from the R component to approximate the curve of depth information obtained from the B component (FIG. 7 below), so that the curve of depth information obtained from the corrected R component can be used in place of the curve of depth information obtained from the B component, and the difference of the depth information obtained from the corrected R component and the depth information obtained from the G component can be measured as thickness from the retina surface to the pigment epithelium of the retina.

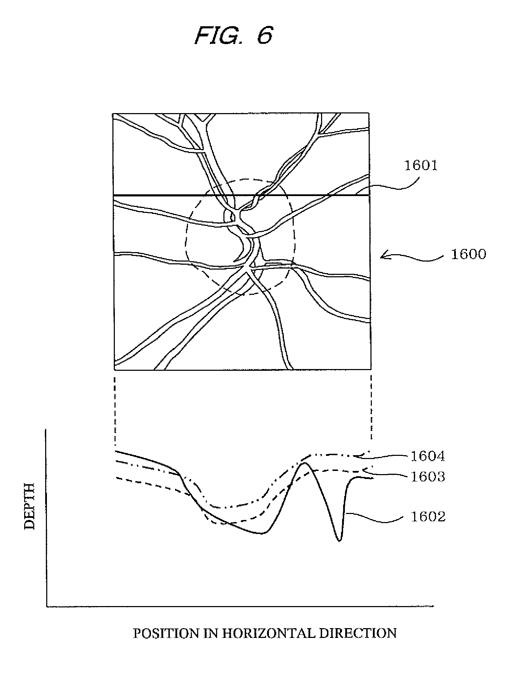

FIGS. 6 and 7 show examples of depth information obtained from red (R) component image data, green (G) component image data, and blue (B) component image data of left and right images by carrying out image processing including the filtering process shown in FIGS. 2a and 2b.

In FIGS. 6 and 7, the waveforms at the bottom show depth information computed (Steps S103, S104 of FIGS. 2a and 2b) for a region corresponding to pixels along a profile line 1601 that is set so as to cut horizontally across an above ocular fundus image 1600 (only the right or left image is shown) photographed by the camera 101. The horizontal axis shows the horizontal (X) direction of the ocular fundus image 1600.

FIG. 6 shows depth information computed (Steps S103, S104 of FIGS. 2a and 2b) from red (R) component image data, green (G) component image data, and blue (B) component image data of left and right images without any filtering process (Step S102 of FIGS. 2a, 2b) being carried out using, e.g., a user setting process or the like. As mentioned previously, the R component is considered as reflected light containing plentiful information from a relatively deep part of the retina, e.g., the choroid, the G component as reflected light containing plentiful information from the pigment epithelium of the retina, and the B component as reflected light containing plentiful information from the retina surface. Assuming this, it is to be expected that curves of depth information computed from left and right images of the color components will not intersect. However, in cases where the filtering process is not carried out, intermixing of reflected light (FIG. 5), for example, from the retina surface causes the curve 1602 of depth information obtained from the red (R) component image data to intersect the curves 1603, 1604 of depth information computed from the other green (G) component image data and the blue (B) component image data, as is shown in FIG. 6. In this case, the depth information obtained from the red (R) component image data cannot be used to derive the thickness of retinal tissue of the ocular fundus.

On the contrary, FIG. 7 shows computation results of depth information in a case where the filtering process (Step S102) of FIGS. 2a, 2b was carried out. In FIG. 7, a curve 1702 of depth information obtained from the red (R) component image data is shown by a single waveform, which represents depth information computed from the red (R) high-frequency component image data in FIG. 2b.

As shown in FIG. 7, when the filtering process of FIGS. 2a, 2b (Step S102) is carried out, curves 1702, 1703, 1704 of depth information computed from the red (R) component image data, the green (G) component image data, and the blue (B) component image data no longer intersect. Furthermore, the red (R) component image data approximates the depth information computed from the blue (B) component image data. Accordingly, these depth information curves 1702, 1703, 1704 can be used to acquire thickness of retinal tissue of the ocular fundus.

Particularly in cases where the above-described filtering process has been carried out, the curve of depth information obtained from the R component approximates the curve of depth information obtained from the B component (FIG. 7 below). Accordingly, the curve of depth information obtained from the corrected R component can be used in place of the curve of depth information obtained from the B component. This allows the difference of the depth information obtained from the corrected R component and the depth information obtained from the G component to be determined as thickness from the retina surface to the pigment epithelium of the retina. In the case where the curve of depth information obtained from the R component having undergone the above-described filtering is used in place of the curve of depth information obtained from the B component, retina thickness can be computed more accurately than using the B component which is prone to errors arising under illumination with a low amount of light. As will be described in a second embodiment to be described below, a problem with image data of the B component is that errors are prone to arise under illumination with a low amount of light. However, if the curve of depth information obtained from the R component having undergone the above-described filtering is used in place of the curve of depth information obtained from the B component, the effect of such errors will be minimal despite the low amount of light of photographing illumination, allowing the accurate computation of retina thickness.

As described above, according to the present embodiment, the left and right parallax images of the stereo-photographed ocular fundus of an eye under examination undergoes color separation to provide stereo images of different frequency components, from which three-dimensional information of ocular fundus tissue, in particular information relating to depth thereof can be derived. Furthermore, computation of differences of depth information derived from the stereo images of the frequency components allows information relating to ocular fundus tissue, in particular to layer thickness of the retina to be acquired. In this case, a predetermined filtering process (elimination or suppression of the high range or low range of spatial frequency), for example, selective extraction of light is performed on a specific wavelength component (in the above-described example, the red (R) component image data). This causes the effect of errors of depth information obtained from the wavelength component to be eliminated, thus allowing depth information relating to a region of tissue associated with the wavelength component to be acquired accurately. This further allows information relating to ocular fundus tissue, in particular to layer thickness of the retina to be acquired accurately.

### <Example of Output for Display>

A display format will be described below which is suitable for output of fundus images obtained by stereo-photographing, or of depth information or information relating to tissue thickness derived from ocular fundus image data in the present embodiment.

FIGS. 8 and 9 show examples of output of measurement results that can be displayed on the display 107 in the ophthalmic measurement apparatus of the present embodiment. Here, examples of output of measurement results are shown primarily using image signals without filtering process.

In the display example of FIG. 8, an ocular fundus image 1800 (a stereo image, or either a left or a right image) is displayed at upper left, and profile lines are displayed along the X (horizontal) and Y (vertical) directions in this fundus image 1800 at positions settable with a mouse or other pointing devices.

In the lower right part of the screen are disposed graphic user interfaces 1803, 1804 comprising radio buttons, buttons operable by a pointing device, or the like. The graphic user interface 1803 is used to select either the left or right stereo image as the image for display as the ocular fundus image 1800; to select an image of any of the R, G, B color components; to select whether to use a pseudo-color map display; and the like.

In the graphic user interface 1804 are disposed radio buttons for selecting which data is used for graphic displays 1801 and 1802 on the lower side and the right side of the ocular fundus image, and buttons such as "OK" and "Cancel" for deciding the selected state of the graphic user interfaces 1803, 1804. In particular, "Color," "Depth," and "Thickness" can be selected on the left side of the graphic user interface 1804. Of these, "Depth" shown in the selected state specifies that the depth information described earlier be displayed, whereas "Thickness" specifies that thickness information be displayed as in FIG. 9 (described below) respectively. "Color" does not specify depth information, rather specifying that information of an image signal, for example, luminance along a profile line be displayed graphically.

The center and right side of the graphic user interface 1804 are for specifying that depth information of any of the color components R, G, and B be used in a subtraction operation to compute "Thickness," i.e., depth information. However, in a state as shown in FIG. 8 in which "Depth" has been specified, there is no direct relationship with the display state.

A "3D Image" button on the lower left of the graphic user interface 1804 is for specifying display of a stereo-photographed three-dimensional image. While the display format of this three-dimensional display is not described in the present embodiment, any of the display formats known in the art can be used.

In the state of FIG. 8, the graphic user interface 1803 displays the left image, and it has been selected to carry out display of "RGB," i.e. of a color image, as the image. Additionally, "Depth" has been selected in the graphic user interface 1804. This selection causes the graphic displays 1801 and 1802 to be outputted on the lower side and right side of the ocular fundus image 1800 to provide a graphic representation of depth information taken respectively along profile lines in the X and Y directions of the ocular fundus image 1800.

Here, the selection is made in the graphic user interface 1803 so as to carry out display of "RGB," i.e. of a color image. Accordingly, the graphic displays 1801 and 1802 represent depth information of three waveforms derived from the left and right R, G, and B color components. In the state of FIG. 8 the depth information is displayed in a state in which a filtering process has not been carried out, as in the case of FIG. 6, and portions of the depth information waveforms intersect.

On the other hand, FIG. 9 shows a screen having graphic user interfaces 1903, 1904 comparable to those in FIG. 8. The left image and color display (RGB) are specified in the graphic user interface 1903, and display of "Thickness" (thickness display) has been selected from the graphic user interface 1904. In the state of FIG. 9, in the center and right side of the graphic user interface 1904 it is specified to subtract the depth obtained from the B component from the depth obtained from the G component. Additionally, display of a pseudo-color map has been selected as well in the graphic user interface 1903. These settings cause the graphic displays 1901, 1902 to be carried out on the lower portion and right of the ocular fundus image 1900.

The stereo-photographic data of FIG. 9 is similar to that of FIG. 8. Thickness values have been derived by subtracting the depth obtained from the B component from the depth obtained from the G component in a state in which a filtering process has not been carried out, as in the case of FIG. 6. As a result, some of the numerical values for measured thickness are negative, and this is particularly remarkable in the graphic display 1902 in the Y direction.

In FIG. 9, display of a pseudo-color map has been selected from the graphic user interface 1903. This display of a pseudo-color map is carried out using an opaque display superimposed over the ocular fundus image 1900 across the entire screen for the purpose of display with different colors, for example, in dependence on the magnitude of numerical values of thickness (in the case where display of a pseudo-color map has been selected in FIG. 8, numerical values of depth). In this case, the pseudo-color map is color-arranged such that density (chroma) increases as numerical values of thickness (or depth) increase.

With such display of the pseudo-color map, regions such as those shown in part by reference numerals 1910 and 1911, particularly regions in which numerical values of thickness are extremely small (e.g., negative values) or extremely large in the graphic display 1902 are displayed with corresponding density (chroma) at the end portions of the pseudo-color map display. Therefore, the examiner can recognize such abnormalities (abnormalities in retinal tissue of an eye under examination, or abnormalities in measurement) at a glance.

While FIGS. 8 and 9 show display results in the absence of a filtering process, it shall be apparent that some other user interface can be provided which allows depth data and thickness data obtained via a filtering process to be displayed using the user interface as shown in FIGS. 8 and 9. In this case, depth data, i.e. depth data obtained from the images of the color components will be displayed, as shown in FIG. 7, as curves substantially corresponding to depth of the layers of retinal tissue.

A different embodiment of an image processing routine different from that shown in FIGS. 2a and 2b is shown below.

### Embodiment 2

In the present embodiment, there is shown an example of image processing suitable for a case in which the G component is treated as reflected light containing plentiful information from the pigment epithelium of the retina and the B component as reflected light containing plentiful information from the retina surface, and thickness information from the retina surface to the pigment epithelium of the retina is derived from the difference in depth information respectively obtained from images of these wavelength components. In the present embodiment, configurations not described explicitly hereinbelow, such as the hardware configuration and the like, are comparable to the configurations used in Embodiment 1.

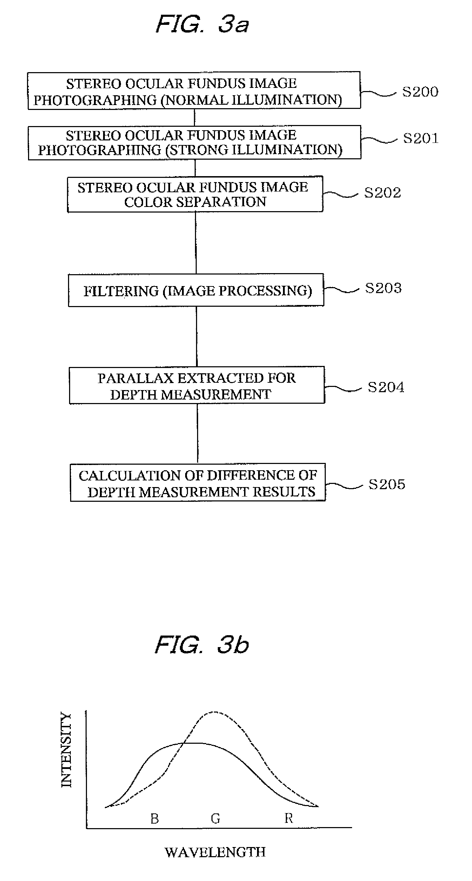

FIG. 3b is a graph illustrating image processing in the present embodiment, and shows wavelength on the horizontal axis and image signal intensity (luminance) obtained by the imaging element of the camera 101 on the vertical axis. The broken line in FIG. 3b shows intensity (luminance) of an image signal typically obtained by the imaging element of the camera 101. A sensitivity distribution in relation to wavelength in the imaging element such as the CMOS, CCD causes a greater luminance distribution to be obtained in the green (G) component image data than in the red (R) component image data or the blue (B) component image data.

The blue (B) component image data, which is considered to be largely reflective of image information of tissue close to the surface of the retina, is susceptible to the effects of noise due to surface reflection and the like. Therefore, such effects of noise would be reduced if an image signal of the greatest possible intensity (luminance) can be obtained. For example, if an image signal is obtained which has an intensity (luminance) distribution of illumination light in which the amount of light is stronger for the blue (B) component than for the other components as shown by the solid line, accurate depth and thickness information for ocular fundus tissue would be obtained owing to reduced effects of noise due to surface reflection and the like.

Thus, according to the present embodiment, photographing of stereo fundus images is carried out twice, at normal illumination intensity and at strong illumination intensity (Steps S200, S201 described below). An image obtained at strong illumination intensity is used for an image of a specific wavelength component, in particular, the blue (B) component, and images obtained at normal illumination intensity are used for images of the other wavelength components. This provides an effect substantially like that when an image signal is used which has an intensity (luminance) distribution such as that obtained with the solid line of FIG. 3b.

FIG. 3a shows an example of an image processing routine different from that of Embodiment 1. FIG. 3a shows the image processing routine of the present embodiment in a format equivalent to that of FIGS. 2a and 2b of Embodiment 1. A program for the CPU 102 to carry out image processing as the principal processor of the image processing apparatus 100 is stored in the ROM 103 or the HDD 105.

In Steps S200 and S201 of FIG. 3a, photographing of stereo ocular fundus images is carried out at normal illumination intensity and at strong illumination intensity, respectively. Steps S202, S203, S204, and S205 are, respectively, a stereo ocular fundus image color separation process, a filtering process, a depth information measurement process, and a thickness information measurement process respectively analogous to Steps S101, S102, S103, and S104 of FIGS. 2a and 2b. Of these, as in Embodiment 1 described previously, the filtering process (S203) can be disabled through a specific setting operation. Additionally, as in Embodiment 1 described previously, the midrange of the spatial frequency may be eliminated or suppressed in order to reduce the effects of errors of depth information in the red (R) component image data.

The depth information measurement process of Step S204 is carried out respectively for the red (R) component image data, the green (G) component image data, and the blue (B) component image data. However, in the present embodiment, at least in the depth information measurement process based on the blue (B) component image data, the depth information measurement process is carried out using blue (B) component image data obtained at strong illumination intensity (Step S201), whereas in the depth information measurement process based on other color (G, R) component image data, the depth information measurement process is carried out using color (G, R) component image data obtained at normal illumination intensity (Step S200). As shall be apparent, in relation to the color components, depth information measurement processes may be carried out respectively both for color component image data obtained at normal illumination intensity (Step S200) and for color component image data obtained at strong illumination intensity (Step S201), so that the data can be used for the purpose of specific measurement.

According to the present embodiment, green (G) component image data obtained at normal illumination intensity (Step S200) and blue (B) component image data obtained at strong illumination intensity (Step S201) are used in the thickness information measurement process of Step S205, and the difference of the two may be derived to provide thickness information from the retina surface to the pigment epithelium of the retina.

Such processing can provide an effect substantially like that when an image signal of intensity distribution such as that shown in FIG. 3b is used, and accurate depth and thickness information for ocular fundus tissue can be obtained owing to reduced effects of noise due to surface reflection and the like.

### Embodiment 3

As shown in FIGS. 5 to 9, blood vessel images, and thick blood vessel images in particular, contained in ocular fundus images can at times hamper depth measurements, possibly giving rise to errors. According to the present embodiment, in order to avoid this problem, the stereo ocular fundus image color separation process, the filtering process, the depth information measurement process, and the thickness information measurement process are carried out after having first eliminated blood vessel images from the stereo-photographed fundus image. Specifically, in the present embodiment, for the depth information measurement process and the thickness information measurement process stereo images are used on which a process to eliminate blood vessel images has been carried out by way of a pre-process. In the present embodiment, the hardware configuration and the like not described explicitly hereinbelow are comparable to the configurations used in Embodiment 1.

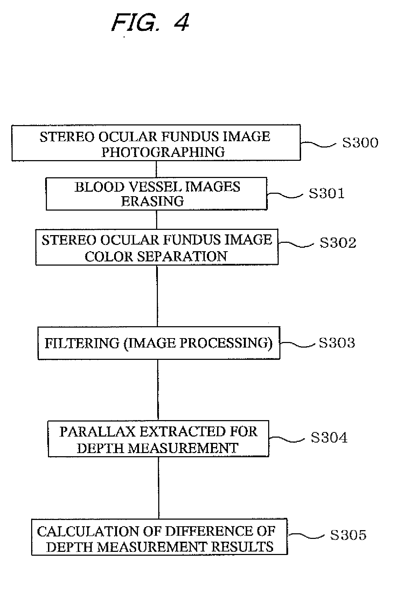

FIG. 4 shows the image processing routine of the present embodiment having a format comparable to that of the above-described FIGS. 2a, 2b, and 3a. A program for the CPU 102 to carry out image processing as the principal processor of the image processing apparatus 100 is stored in the ROM 103 or the HDD 105.

In Step S300 of FIG. 4, photographing of a stereo ocular fundus image is carried out analogously to Embodiment 1 (Step S101 of FIGS. 2a, 2b.)

In Step S301, morphology processing or the like is used to eliminate blood vessel images (preferably thick blood vessel images in particular) from the stereo-photographed ocular fundus image.

The subsequent Steps S302, S303, S304, and S305 are respectively a stereo ocular fundus image color separation process, a filtering process, a depth information measurement process, and a thickness information measurement process respectively analogous to Steps S101, S102, S103, and S104 of FIGS. 2a and 2b. The stereo ocular fundus image from which blood vessel images were eliminated in Step S301 is used as the input to these processes. Of these, as in Embodiment 1 described previously, the filtering process (S303) can be disabled through a specific setting operation. Additionally, as in Embodiment 1 described previously, the high range or low range of the spatial frequency may be eliminated or suppressed in order to reduce the effects of errors of depth information in the red (R) component image data.

In the thickness information measurement process of Step S305, depth information obtained from component image data of any wavelength from among the red (R) component image data, the green (G) component image data, and the blue (B) component image data can be selected, and the thickness of the retinal tissue can be measured and outputted by carrying out a subtraction process, as is similar to the above-described Embodiments 1 and 2.

As described above, in the present embodiment, blood vessel images are first eliminated from the stereo-photographed ocular fundus image to carry out the stereo ocular fundus image color separation process, the filtering process, the depth information measurement process, and the thickness information measurement process. This allows errors to be reduced which may arise due to blood vessel images contained in the ocular fundus image in depth measurement and hence in thickness measurement carried out on the basis thereof.

While examples of minimum configurations for solving the problem are shown in the above-described embodiments, a pre-process may be added in which images having undergone color separation are subjected to a process for correcting color aberration.

### Industrial Applicability

The present invention can be implemented in image processing apparatuses such a fundus camera, an ophthalmic measurement device, a filing device, or the like for carrying out image processing for outputting, for display, ocular fundus images of an eye under examination.

### Key to Symbols

- 100: image processing apparatus
- 101: camera
- 102: CPU
- 103: ROM
- 104: HDD
- 106: network
- 107: display
- 402, 401, 1600, 1800, 1900: ocular fundus images
- 1601: profile line
- 1903, 1904: graphic user interface

## Claims

1. An image processing method in which an ocular fundus of an eye under examination is stereo-photographed with a predetermined parallax via an ocular fundus photographing optical system to provide left and right parallax images, which are used for processes of measuring a three-dimensional shape of the ocular fundus of the eye under examination, comprising:
subjecting the photographed stereo ocular fundus images to color separation;
performing a depth information measurement process in which depth information at a specific ocular fundus region is derived for each of the stereo ocular fundus images of different wavelength components obtained by the color separation, and
performing a thickness information measurement process in which a difference in the depth information obtained respectively in the depth information measurement process from the stereo ocular fundus images of different wavelength components is derived as thickness information for specific ocular fundus tissue.

2. An image processing method according to claim 1, wherein a process of filtering relating to spatial frequency is performed for at least any one of the images of different wavelength components obtained by the color separation.

3. An image processing method according to claim 1, wherein the color separation is performed so as to provide red (R) component image data, green (G) component image data, and blue (B) component image data, a process of filtering relating to spatial frequency being performed on the red (R) component image data, and, in a case where the red (R) component image data is to be used in the depth information measurement process and the thickness information measurement process, a high-frequency component of the red (R) component image data and a low-frequency component of the red (R) component image data are used.

4. An image processing method according to claim 1, wherein, in the depth information measurement process and the thickness information measurement process, the red (R) component is treated as reflected light including information from relatively deep part of the retina, for example, from the choroids and information from the retina surface; the green (G) component as reflected light including plentiful information from the pigment epithelium of the retina; and the blue (B) component as reflected light including plentiful information from the retina surface.

5. An image processing method according to claim 1, wherein the stereo-photographing is performed a plurality of times using different amounts of illumination light, and, in the depth information measurement process and the thickness information measurement process, an image that is photographed using an amount of illumination light that is different from images of other wavelength components is used for at least any one of the images of different wavelength components obtained by the color separation.

6. An image processing method according to claim 5, wherein as the blue (B) component image an image is used which is photographed using an amount of illumination light that is stronger than for images of other color components in order to make the amount of light stronger for the blue (B) component than for the other components in a wavelength distribution of the amount of illumination light.

7. An image processing method according to claim 1, wherein stereo images on which a process for eliminating blood vessel images is performed as a pre-process are used in the depth information measurement process and the thickness information measurement process.

8. An image processing apparatus, including an ocular fundus photographing optical system and image processing means for carrying out the image processing method according to any of claims 1 to 7.
